# EUROPÄISCHE PATENTSCHRIFT

(11) **EP 2 925 211 B1**
(45) Veröffentlichungstag und Bekanntmachung des Hinweises auf die Patenterteilung: **21.02.2024**
(21) Anmeldenummer: 13798992.7
(22) Anmeldetag: 26.11.2013
(51) Int. Cl.: A61B 5/16

(54) **VERFAHREN ZUR ÜBERPRÜFUNG DER VALIDITÄT VON REAKTIONEN EINER PERSON**
METHOD FOR CHECKING THE VALIDITY OF REACTIONS OF A PERSON
PROCÉDÉ DE VÉRIFICATION DE LA VALIDITÉ DES RÉACTIONS D'UNE PERSONNE

(30) Priorität: 03.12.2012 DE 102012111733
(43) Veröffentlichungstag der Anmeldung: 07.10.2015
(73) Patentinhaber: NPAOE GmbH & Co. KG, 71083 Herrenberg (DE)
(72) Erfinder: GLAUSINGER, Klaus, 72076 Tübingen (DE)
(74) Vertreter: Mammel und Maser Patentanwälte PartG mbB
(86) Internationale Anmeldenummer: PCT/EP2013/074722
(87) Internationale Veröffentlichungsnummer: WO 2014/086618

(56) Entgegenhaltungen:
- US-A- 5 957 859
- DONG GUANGHENG ET AL: "The presentation order of cue and target matters in deception study", BEHAVIORAL AND BRAIN FUNCTIONS, BIOMED CENTRAL, LONDON, GB, Bd. 6, Nr. 1, 22. Oktober 2010 (2010-10-22), Seite 63, XP021080772, ISSN: 1744-9081, DOI: 10.1186/1744-9081-6-63
- MOHAMMADIAN A ET AL: "Multimodal Detection of Deception using Fusion of Reaction Time and P300 Component", BIOMEDICAL ENGINEERING CONFERENCE, 2008. CIBEC 2008. CAIRO INTERNATIONAL, IEEE, PISCATAWAY, NJ, USA, 18. Dezember 2008 (2008-12-18), Seiten 1-4, XP031425981, ISBN: 978-1-4244-2694-2
- LEE T M C ET AL: "Are errors differentiable from deceptive responses when feigning memory impairment? An fMRI study", BRAIN AND COGNITION, ACADEMIC PRESS, NEW YORK, NY, US, Bd. 69, Nr. 2, 1. März 2009 (2009-03-01), Seiten 406-412, XP025915411, ISSN: 0278-2626, DOI: 10.1016/J.BANDC.2008.09.002 [gefunden am 2008-10-19]
- RAY JOHNSON ET AL: "The contribution of executive processes to deceptive responding", NEUROPSYCHOLOGIA, Bd. 42, Nr. 7, 1. Januar 2004 (2004-01-01) , Seiten 878-901, XP55103181, ISSN: 0028-3932, DOI: 10.1016/j.neuropsychologia.2003.12.005
- ZORDAN ET AL: "ERP components activated by the ''GO!'' and ''WITHHOLD!'' conflict in the random Sustained Attention to Response Task", BRAIN AND COGNITION, ACADEMIC PRESS, NEW YORK, NY, US, vol. 66, no. 1, 29 January 2008 (2008-01-29), pages 57-64, XP022438289, ISSN: 0278-2626, DOI: 10.1016/J.BANDC.2007.05.005

## Beschreibung

Die Erfindung betrifft ein Verfahren zur Prüfung der Echtheit der von einem Probanden erzielten Reaktionszeit.

Bei testpsychologischen Untersuchungen von Probanden handelt es sich in vielen Fällen um eine gutachterliche Beurteilung im entschädigungsrelevanten Kontext (Unfallversicherungen, Berufs- und Dienstunfähigkeit etc.). Hierfür sind Kenntnisse über die Anstrengungsbereitschaft und Validität der geltend gemachten Beschwerden - also die Bereitschaft der Probanden, vollständig und wahrheitsgemäß, mit vollem geistigen Einsatz und ohne Vortäuschung falscher Tatsachen Angaben zu machen oder vorgegebene Tests zu bearbeiten - für eine objektive Beurteilung von entscheidender Bedeutung.

Die Überprüfung der Beschwerdevalidität ist für die Diagnosestellung einer psychischen Gesundheitsstörung unabdingbar. Ein wichtiges Kriterium ist hierbei die Messung der *Anstrengungsbereitschaft* des Probanden.

Die Häufigkeit von Täuschungsversuchen ist dabei sehr gut untersucht und beträgt für den deutschsprachigen Raum bis zu 50 %, in den USA liegt der Anteil von Täuschungsversuchen teilweise noch höher. Der Schaden für die Sozialgemeinschaft durch nicht erkannte Täuschungen ist folglich immens.

Stand der Technik für die überwiegende Mehrheit der Testverfahren zur Erfassung der Beschwerden eines Probanden sind papiergebundene Selbstbeurteilungsfragebögen, die manuell vom Probanden ausgefüllt und unter Nutzung von Lösungsbögen ausgewertet werden. Zur Testung kognitiver Funktionen stehen zahlreiche Testverfahren, sowohl papiergebunden als auch vereinzelt am PC, zur Verfügung.

Die Überprüfung der Anstrengungsbereitschaft und Beschwerdevalidität der Probanden kann heute durch mehrere separate Testverfahren neben der eigentlich durchgeführten Testung betrieben werden. Dabei werden zum einen augenscheinlich valide Testverfahren angewandt, die vorgeben, kognitive Funktionen zu überprüfen, in Wirklichkeit aber sehr einfache, auch von beispielsweise hirngeschädigten und dementen Probanden zu lösende Aufgaben beinhalten. Deren Ergebnisauswertung erlaubt dann eine Aussage über die Anstrengungsbereitschaft der untersuchten Probanden. Zum anderen bedienen sich die gängigen Verfahren einer Überprüfung der Antwortkonsistenzen, die Aussagen über das Testverhalten erlaubt. Die Überprüfung der Beschwerdevalidität kann zusätzlich mittels Fragebogen erfolgen. Hierbei werden neben echten Symptomen abwegige Symptome abgefragt und anhand von Summenscores eine Aussage darüber getroffen, inwiefern die Beschwerden authentisch erscheinen.

Die allgemeinen Empfehlungen der Fachgesellschaften lauten dahingehend, dass immer mehrere Verfahren zum Einsatz kommen sollen. Die abschließende Bewertung der Testleistungen obliegt dem Gutachter/Sachverständigen.

Die bisher etablierten Verfahren decken nur einen sehr kleinen Teil der Überprüfungs- und Validierungsmöglichkeiten ab, in dem Beschwerdeverdeutlichung betrieben werden kann. Grundsätzlich ist es leicht, Defizite aus allen körperlichen Funktionsbereichen vorzutäuschen. Verfahren zur Aufdeckung von Simulation oder Aggravation bei körperlichen (z.B. neurologischen) Untersuchungen (wie Gefühlsstörungen) oder bei Aufmerksamkeitstests (direkte objektive Validierung von Reaktionszeiten) existieren bisher weder in der wissenschaftlichen noch in der Patentliteratur.

Aus der US 5,957,859 ist ein Verfahren zur Überprüfung der Validität von Reaktionen einer Person bekannt, bei dem der Proband einem Testreiz ausgesetzt wird und parallel zu der Messung der Reaktion auf diesen Testreiz auch die ereigniskorrelierten Potenziale (ERP) über die Ableitung der Hirnstromkurven des Probanden gemessen werden. Die Werte werden mit den Werten verglichen, die der Proband bei einem Kontrollreiz, der einer ehrlichen Reaktion entsprechen soll, gezeigt hat. Nachteilig an diesem Verfahren ist, dass der Proband die Bewertung durch Manipulation der Reaktion auf den Kontrollreiz verfälschen kann und keine objektivierbare und reproduzierbare technische Möglichkeit existiert, den Wahrheitsgehalt der Reaktion des Probanden auf den Kontrollreiz zu überprüfen.

Verfahren zur Überprüfung der Validität von Reaktionen von Probanden, wobei den Probanden ein Stimulus unter Ableitung von visuell evozierten Potenzialen aus den Hirnstromkurven demonstriert und den Probanden eine Reaktionszeitaufgabe gestellt und die Reaktionszeit gemessen wird, sind bei Dong Guangheng et AI: "The presentation order of cue and target matters in deception study", Behavioral and Brain Functions, Biomed Central, London; GB, Bd. 6, Nr. 1, 22. Oktober 2010 (2010-10-22), Seite 63, XP021080772, ISSN: 1744-9081, DOI: 10.1186/1744-9081-6-63 und von Lee T M C et AI: "Are errors differentiable from deceptive responses when feigning memory impairment? An fMRI study", Brain and Cognition, Academic Press, New York, NY, US, Bd. 69, Nr. 2, 1. März 2009 (2009-03-01), Seiten 406-412, XP025915411, ISSN: 0278-2626, DOI: 10.1016/J. BANDC.2008.09.002 beschrieben.

Verfahren zur Überprüfung der Validität von Reaktionen von Probanden sind darüber hinaus auch bei Lee T M C et Al: "Are errors differentiable from deceptive responses when feigning memory impairment? An fMRI study", Brain and Cognition, Academic Press, New York, NY, US, Bd. 69, Nr. 2, 1. März 2009 (2009-03-01), Seiten 406-412, XP025915411, ISSN: 0278-2626, DOI: 10.1016/J. BANDC.2008.09.002 [gefunden am 2008-10-19] und bei Ray Johnson et Al: "The contribution of executive processes to deceptive responding", Neuropsychologia, Bd. 42, Nr. 7, 1. Januar 2004 (2004-01-01), Seiten 878-901, XP55103181, ISSN: 0028-3932, DOI: 10.1016/j. neuropsychologia.2003.12.005 Abschnitte "Methods" und "Results", 3.2 dargestellt. In der Veröffentlichung "ERP components activated by the "Go!" and "Withhold!" conflict in the random sustained attention to response task" von Lara Zordan et. al in "Brain and Cognition", Academic Press, New York, Bd. 66, Nr. 1, 20. Januar 2008, Seite 57 - 64 wird untersucht, wie sich bestimmte Komponenten von ereigniskorrelierten Potenzialen verändern, wenn eine motorische Reaktion ganz unterdrückt wird ("Go-Nogo-Paradigma").

Die Aufgabe der Erfindung besteht darin, ein Verfahren zur Verfügung zu stellen, das es ermöglicht, reproduzierbar die Prüfung der Echtheit der von einem Probanden erzielten Reaktionszeit durchzuführen.

Diese Aufgabe wird durch die Merkmale des Anspruchs 1 gelöst.

Bei dem erfindungsgemäßen Verfahren erfolgt die Prüfung der Echtheit der von einem Probanden erzielten Reaktionszeit dadurch, dass dem Probanden ein Stimulus unter Ableitung der evozierten Potenziale (EP) aus der Hirnstromkurve oder unter Darstellung der Hirnareale mittels funktioneller Bildgebungsverfahren demonstriert wird und dem Probanden eine Reaktionszeitaufgabe gestellt und die Reaktionszeit unter Ableitung der Bereitschaftspotenziale gemessen wird.

Bei dem erfindungsgemäßen Verfahren wird über die Ableitung der evozierten Potenziale aus der Hirnstromkurve oder Darstellung der Aktivität der Hirnareale mittels funktioneller Bildgebungsverfahren (z.B. NIRS (Nahinfrarotspektroskopie) oder fMRT (funktionelle Magnetresonanztomographie) bei der Demonstration des Stimulus überprüft, ob der Stimulus in das Bewusstsein des Probanden gelangt ist. Damit steht sowohl eine elektrophysiologische als auch eine bildgebende Methode bereit, die reproduzierbar ist.

Die Ableitung von evozierten Potenzialen ist eine reproduzierbare elektrophysiologische Methode. Vorzugsweise werden visuell evozierte Potenziale (VEP), AEHP (akustisch evozierte Hirnstammpotenziale), somatosensibel evozierte Potenziale (SEP) oder durch Riechreize evozierte Potenziale abgeleitet.

Zeitgleich wird dem Probanden eine Reaktionszeitaufgabe gestellt, vorzugsweise derart, dass der Proband auf einen Reiz umgehend reagieren soll, und die Reaktionszeit unter Ableitung der Bereitschaftspotenziale gemessen wird.

Unter Bereitschaftspotenzialen werden im Rahmen der vorliegenden Erfindung insbesondere die motorischen und/oder lateralisierten Bereitschaftspotenziale (RP und/oder LRP) verstanden. Die LRP werden dabei sowohl stimulusbezogen (sLRP) als auch reaktionsbezogen (LRPr) ermittelt. Dies bietet den Vorteil, dass Prozesse, die vor der selektiven Aktivierung involviert sind (d. h. Prozesse im Intervall zwischen der Präsentation des Stimulus und dem Auftreten des LRP) von nachfolgenden Prozessen (d. h. Prozesse im Intervall zwischen dem Auftreten des LRP und dem Einsetzen der beobachtbaren Reaktion) unterschieden werden können (Eimer, M. (1998). The lateralized readiness potential as an online measure of central response activation processes. Behaviour Research Methods, Instruments & Computers, 30, S. 146-156. und Mordkoff, J. T., Miller, J. & Roch, A. (1996). Absence of coactivation in the motor component : Evidence from psychophsysiological measures of target detection. Journal of Experimental Psychology: Human Perception and Performance, 22, S. 25-41.). Die vorliegende Erfindung beseitigt dadurch den Mangel im Stand der Technik, als bisher lediglich stimulusbezogene ERP zur Beurteilung herangezogen wurden, die um eine Reaktion ableitbaren Bereitschaftspotenziale aber unberücksichtigt blieben. Durch die vorliegende Erfindung werden also Sensitivität und Spezifität erhöht.

Eine Korrelation erfolgt derart, dass die Absolutwerte der Reaktionszeiten und deren Varianzen in Bezug zu den Bereitschaftspotenzialen gesetzt werden. In einem zweiten Schritt der Bewertung werden die Bereitschaftspotenziale (Amplituden und Latenzen) mit den aus anderen Untersuchungen bekannten Werten abgeglichen.

Erfindungsgemäß kann eindeutig eine Unterscheidung zwischen einer bewussten Verzögerung und einer tatsächlichen Verzögerung aufgrund einer Hirnfunktionsstörung getroffen werden. Hierdurch kann die Anstrengungsbereitschaft von Probanden ermittelt und bewertet werden.

Bevorzugt werden erfindungsgemäß lateralisierte Bereitschaftspotenziale abgeleitet, da hierdurch die bei der Auswertung der ERP auftretenden Verfälschungen durch andere Potenziale (potenzielle Seitenfehler) vermieden werden können.

Nachdem bei dem erfindungsgemäßen Verfahren über die Auswertung der Hirnströme oder der Bildgebungsverfahren zunächst überprüft wird, ob der Stimulus in das Bewusstsein gelangt, und das erfindungsgemäße Verfahren bei der Beurteilung und Auswertung zudem berücksichtigt, wie das LRP-Verhalten von gesunden Probanden, die mit voller Leistungsbereitschaft reagieren bzw. die Antwort bewusst verfälschen, und tatsächlich hirngeschädigten, z.B. dementen, oder psychisch kranken, Probanden ist, kann die im Stand der Technik für eine korrekte Auswertung erforderliche wahrheitsgemäße Reaktion auf den Kontrollreiz entfallen. Dies erfolgt dadurch, dass neben der elektrophysiologischen Ableitung der genannten evozierten Potenziale und/oder bildgebend aquirierten Aktivitätsniveaus der entsprechenden Hirnareale ein Abgleich mit eigens in umfangreichen klinischen Studien erhobenen Daten der genannten Bereitschaftspotenziale von gesunden mit voller Leistungsbereitschaft reagierenden Probanden, gesunden, die Antwort verfälschenden Probanden und Probanden mit Hirnfunktionsstörung durchgeführt wird.

Die durch das Voraussetzen des Bewusstseinwerdens der Stimuli und durch Manipulationen beim Kontrollreiz auftretenden Probleme im Stand der Technik werden somit vermieden.

In einer bevorzugten Ausführungsform wird zusätzlich zu der gleichzeitigen Ableitung der LRP, und der evozierten Potenziale sowie zusätzlich Anwendung funktioneller Bildgebungsverfahren auch die Aktivität der Zielmuskulatur abgeleitet und dies mit in die Bewertung einbezogen. Dies erfolgt derart, dass vorzugsweise durch eine Oberflächenableitung aus den für die Reaktion erforderlichen Muskeln die Muskelaktivität (EMG, Elektromyogramm) gemessen wird.

Hierdurch wird eine Detektion einer möglichen Vorinnervation der Zielmuskulatur vor der sichtbaren und vorzugsweise motorischen Reaktion erreicht. Durch Abgleich mit den ermittelten Reaktionszeiten und den oben genannten aus der Hirnstromkurve resultierenden Potenzialen und/oder den Ergebnissen der funktionell bildgebenden Verfahren gelingt so die Feststellung, ob eine motorische Reaktion bereits bewusst initiiert war, deren Effekt aber absichtlich noch zurückgehalten wurde.

Das Verfahren kann sich somit fakultativ zudem der Messung der Muskelaktivität der für die Reaktion erforderlichen Muskulatur bedienen, wodurch eine funktionell wirkungslose Vorinnervation (nicht sichtbare Reaktion) - im Gegensatz zum Stand der Technik - erfasst werden kann.

Ein weiterer Vorteil der vorliegenden Erfindung besteht darin, dass diese auch Hirnstromantworten von tatsächlich Kranken berücksichtigt. Dies ist bei den bislang bekannten Verfahren nicht der Fall, da die Ursache von Falschantworten, die beispielsweise auch eine Hirnschädigung im dorsolateralen präfrontalen Cortex ("Lügenzentrum") sein könnte, bislang nicht berücksichtigt wurde.

Vorzugsweise erfolgt die Ableitung nach dem international anerkannten 10-20-System an mindestens acht Orten. Durch die multilokuläre Ableitung werden die bei zentralen Ableiteorten auftretenden Probleme wie das Unberücksichtigtbleiben der physiologischen Seitenunterschiede (z.B. Problem der dominanten Hemisphäre: bei Rechtshändern liegen die Sprachzentren links und umgekehrt, im Verlauf von Bereitschaftspotenzialen auftretende Lateralisierung) vermieden. Außerdem resultiert aus der Feldaufzeichnung durch multilokuläre Ableitung der genannten Hirnstrompotenziale eine höhere Validität der genannten Potenziale dadurch, dass physiologische oder krankhafte Hirnstromkurvenveränderungen detektiert und in der Bewertung der Potenziale berücksichtigt werden und somit die im Stand der Technik bestehende Artefaktanfälligkeit behoben wird.

Dadurch, dass bei dem erfindungsgemäßen Verfahren das gesamte Antwortverhalten, somit auch fehlende oder unpassende Antworten, mit in die Bewertung einbezogen werden, ist das Verfahren weitaus zuverlässiger als die bislang bekannten Verfahren, denn es berücksichtigt, dass unpassende oder fehlende Antworten auch physiologisch bedingt und/oder durch Hirnschädigungen bedingt auftreten können.

Um den Einfluss von die Beurteilung möglicherweise negativ beeinflussenden Faktoren möglichst gering zu halten, wird erfindungsgemäß eine sehr einfache Reaktionsaufgabe gestellt, so dass in diese nicht zahlreiche kognitive Prozesse wie bei bislang bekannten Verfahren einfließen.

Das erfindungsgemäße Verfahren ermöglicht auch, die bereits bekannten Testverfahren zu ergänzen.

Das erfindungsgemäße Verfahren sieht vor, von Probanden erzielte Reaktionszeiten unter Einsatz eines EEG (Elektroenzephalogramm) mit Bestimmung der genannten Bereitschaftspotenziale hinsichtlich ihrer Echtheit zu prüfen und diese Daten automatisiert auszuwerten.

Das erfindungsgemäße Verfahren ermöglicht die Prüfung der Echtheit der von einem Probanden erzielten Reaktionszeit auf Basis einer physikalisch nachweisbaren Messung der Hirnströme eines Probanden, und zwar ohne dass - wie bislang - immer hilfsweise eine Konsistenzprüfung zur Beurteilung der Validität herangezogen werden muss. Die bislang erforderliche Konsistenzprüfung bedingte eine erheblich schlechtere Sensitivität und Spezifität als bei dem erfindungsgemäßen Verfahren.

Das Verfahren bedient sich dabei der Messung von Hirnströmen mit dem EEG und/oder der Darstellung der Hirnareale mittels funktioneller Bildgebungsverfahren. Ein wiederkehrender, zum Beispiel visueller, akustischer, elektrischer, taktiler oder olfaktorischer Reiz wird dem zu untersuchenden Probanden gezeigt. Dieser erhält die Aufgabe, auf den Reiz so schnell als ihm möglich zu reagieren und eine Taste zu drücken. Dabei werden auch die in der Routinediagnostik etablierten VEP und/oder AEHP und/oder SEP und/oder olfaktorisch evozierten Potenziale abgeleitet, um zu zeigen, dass der Proband den Stimulus erkennen konnte.

Im Rahmen des erfindungsgemäßen Verfahrens erfolgt vorzugsweise eine automatisierte computerbasierte Auswertung der umfangreichen Messdaten mit einem mathematischen Algorithmus. Die Auswertung und Integration aller Funktionen und Geräte erfolgt auf einem Computer, mit dem ein EEG-Gerät sowie ein Gerät zur Präsentation des Stimulus verbunden werden.

Zuletzt kann durch Erstellung einer Ergebnisbewertungsempfehlung für den Gutachter/Sachverständigen auf Basis der automatisierten Auswertung der Messdaten eine Beurteilungsempfehlung hinsichtlich der Echtheit der von dem Probanden erzielten Reaktionszeit ausgesprochen werden, die ein eindeutiges Unterscheiden von bewusst verzögerten motorischen Handlungen von tatsächlich auf Hirnfunktionsstörungen basierenden Reaktionsverzögerungen ermöglicht. Die Ergebnisbewertungsempfehlung kann hierbei beispielsweise entweder als Papierausdruck oder als digitale Benachrichtigung ausgegeben werden

Im Rahmen der vorliegenden Erfindung werden vorzugsweise Hirnstromkurven der Hirnrinde gemessen, da tiefer liegende Hirnregionen ohne invasive Technik nicht eindeutig messbar sind. Die Hirnströme werden bevorzugt mittels EEG gemessen.

Eine Vorrichtung ist derart gestaltet, dass ein Wiedergabegerät, eine Reaktionsvorrichtung und ein Reaktionszeitmesser zum Einsatz kommen. Außerdem ist ein Rechner umfasst, auf welchem ein mathematischer Algorithmus abläuft und hierbei die Ergebnisse des Wiedergabegeräts, der Reaktionsvorrichtung sowie des Reaktionszeitmessers mit der Auswertung der Hirnstromkurven und/oder der Darstellung der Hirnareale mittels funktioneller Bildgebungsverfahren und des EMGs in eine direkte Relation setzt. Als Stimulusgerät kann beispielsweise ein Audio- und/oder ein visuelles und/oder ein taktiles und/oder olfaktorisches Gerät umfasst sein. Eine Reaktionsvorrichtung kann beispielsweise ein Druckknopf oder ein Joystick sein. Der Reaktionszeitmesser ist derart gestaltet, dass er die Zeit zwischen der Wiedergabe des Audio- und/oder visuellen und/oder taktilen und/oder olfaktorischen Gerätes und dem Betätigen der Reaktionsvorrichtung misst.

Das erfindungsgemäße Verfahren wird nachfolgend in einer möglichen Ausführungsform am Beispiel eines visuellen Stimulus und der Ableitung der LRP näher beschrieben:

Dem Probanden wird erläutert, dass er auf einen visuellen Stimulus so schnell wie möglich reagieren soll ("Reaktionszeitaufgabe").

Die Stimulation erfolgt bei diesem Beispiel durch ein sich in seiner zeitlichen Abfolge zufällig invertierendes Schachbrettmuster. Der Reiz wird in einem Messvorgang 200 Mal appliziert. Die Reaktionszeitaufgabe lautet somit, dass der Proband, sobald er den Reiz in Form einer Invertierung des Schachbrettmusters wahrgenommen hat, reagieren soll. Die Reaktion erfolgt bei diesem Beispiel durch einen Tastendruck.

Der Proband wird an ein EEG angeschlossen und der Reiz wird unter Ableitung der evozierten Potenziale aus den Hirnstromkurven und Messung der Reaktionszeit demonstriert. Die evozierten Potenziale, im vorliegenden Beispiel VEP, werden mittels stimulusgetriggerter Mittelung aus der über den entsprechenden Hirnarealen (im vorliegenden Beispiel bioccipital) abgeleiteten Hirnstromkurve ermittelt. Die Bereitschaftspotenziale werden dabei analog der Ermittlung der VEP - allerdings multilokulär - und zusätzlich reaktionsgetriggert bestimmt. Dabei wird bei reaktionsgetriggerter Mittelung ein Zeitraum von mindestens 500 ms vor der Reaktion und 1200 ms nach erfolgter Reaktion untersucht. Bei stimulusgetriggerter Potenzialmittelung wird ein Zeitraum von mindestens 800 ms nach dem Stimulus erfasst.

Aufgrund dieser Messwerte erfolgt nun die Prüfung der Echtheit der von dem Probanden erzielten Reaktionszeit:

Durch die Ableitung und Vermessung der im vorliegenden Beispiel visuell evozierten Potenziale nach Latenz und Amplitude wird durch Vergleich der ermittelten Werte mit in der Neurologie allgemein anerkannten Normwerttabellen überprüft, ob der Stimulus im Bewusstsein des Probanden angekommen ist, der Proband also den Reiz wahrnehmen konnte.

Zudem werden die LRP - durch reaktions- und stimulusgetriggerte Mittelung (LRPr und sLRP) aus den Hirnstromkurven ermittelt - nach Latenzen und Amplituden vermessen. Sodann erfolgt ein Vergleich mit den bekannten, insbesondere statisch validierten Daten aus den klinischen Untersuchungen an bewusst verzögert und mit voller Leistungsbereitschaft reagierenden gesunden Probanden sowie an hirngeschädigten und psychisch kranken, im genannten Beispiel depressiv erkrankten, Probanden. Dies gelingt aufgrund des Verhaltens der genannten Bereitschaftspotenziale, die sich insbesondere in ihren Amplituden signifikant zwischen den Bedingungen einer vollen Anstrengungsbereitschaft und einer bewusst negativen Antwortverzerrung - auch bei den genannten kranken Probanden - unterscheiden.

Letzteres konnte in einer Untersuchung von je 60 gesunden und depressiv erkrankten Probanden, die neben der oben dargestellten Reaktionszeitaufgabe in einem weiteren Aufgabenteil die Aufforderung um 0.5 Sekunden nach einem Reiz durch Tastendruck zu reagieren (entsprechend einer bewussten Reaktionsverzögerung) erhielten, bestätigt werden: für mehrere Ableiteorte zwischen den beiden Bedingungen fanden sich signifikante Unterschiede der LRP und ERP Amplituden.

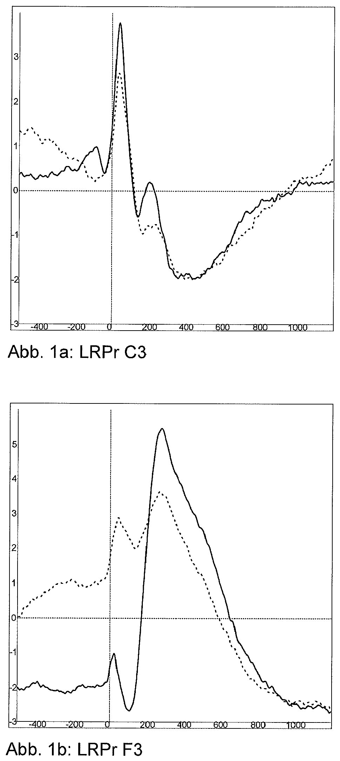

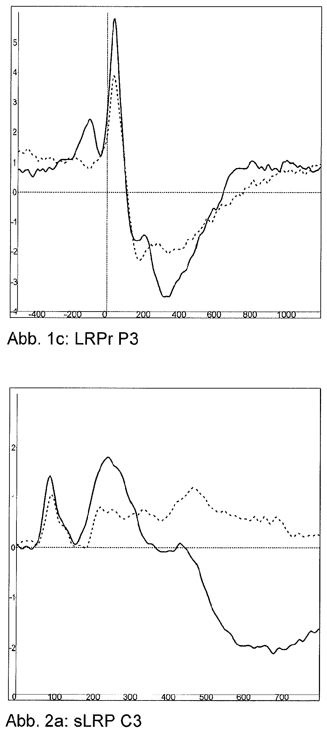

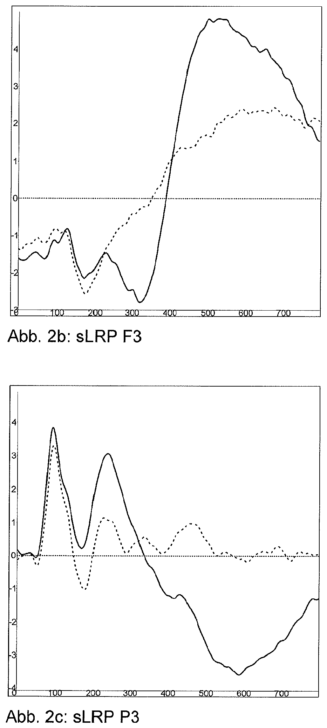

Die Abbildungen 1a-c zeigen beispielhaft die reaktionsgetriggerten, die Abbildungen 2a-c die stimulusgetriggerten LRP von unterschiedlichen Ableiteorten bei gesunden Probanden. Die durchgezogenen Kurven resultieren aus der Anstrengungsbedingung, die gestrichelten aus der Bedingung einer bewussten Reaktionsverzögerung. Das Signifikanzniveau beträgt für alle in den Abbildungen dargestellten Ergebnisse p<0.001. Für alle Abbildungen finden sich auf den y-Achsen die Amplituden in µV, auf den x-Achsen die Zeit in ms.

Für die Ergebnisbewertung im vorliegenden Beispiel wird zusätzlich die Potenzialkonfiguration (zeitlicher Verlauf der Positivitäten und Negativitäten), die je nach Erkrankung unterschiedlich sein kann, in der Bewertung berücksichtigt. Außerdem werden Absolutwerte und Varianzen der Reaktionszeiten mit denen gesunder und mit voller Anstrengungsbereitschaft reagierender Probanden verglichen.

Die Bewertungsempfehlung resultiert also aus einer Prüfung der Echtheit der von einem Probanden erzielten Reaktionszeit und ihrer Streuung, einem Abgleich der Potenziale und ihrer Amplituden mit Ergebnissen der genannten Studien.

## Patentansprüche

1. Verfahren zur Prüfung der Echtheit der von einem Probanden erzielten Reaktionszeit, **dadurch gekennzeichnet, dass** ein Stimulusgerät, eine Reaktionsvorrichtung, ein Reaktionszeitmesser, ein EEG und ein Computer vorgesehen sind und dem Probanden mittels des Stimulusgeräts ein Stimulus unter Ableitung von evozierten Potenzialen aus seinen mittels EEG gemessenen Hirnstromkurven oder unter Darstellung seiner Hirnareale mittels funktionellen Bildgebungsverfahren wie Nahinfrarotspektroskopie oder funktionelle Magnetresonanztomographie fMRT demonstriert wird und dem Probanden zeitgleich mittels der Reaktionsvorrichtung eine Reaktionszeitaufgabe gestellt und mittels des Reaktionszeitmessers die Reaktionszeit des Probanden unter Ableitung seiner Bereitschaftspotenziale gemessen wird, wobei über die Ableitung der evozierten Potentiale aus der Hirnstromkurve oder Darstellung der Aktivitäten der Hirnareale mittels funktioneller Bildgebungsverfahren zunächst überprüft wird, ob der Stimulus in das Bewusstsein des Probanden gelangt ist und wobei die Auswertung mittels des Computers derart erfolgt, dass die Absolutwerte der Reaktionszeiten und deren Varianzen in Bezug zu den Bereitschaftspotenzialen gesetzt werden und die Bereitschaftspotenziale mit den aus anderen Untersuchungen bekannten Werten abgeglichen werden, um die Echtheit der Reaktionszeiten zu prüfen.

2. Verfahren nach Anspruch 1, **dadurch gekennzeichnet, dass** die evozierten Potenziale visuell evozierte Potenziale, VEP, akustisch evozierte Hirnstammpotenziale, AEHP, somatosensibel evozierte Potenziale, SEP unter Einbeziehung von taktil evozierten Potenzialen oder olfaktorisch evozierte Potenziale sind.

3. Verfahren nach Anspruch 1, **dadurch gekennzeichnet, dass** die Bildgebungsverfahren funktionelle Magnetresonanztomographie, fMRT, oder Nahinfrarotspektroskopie, NIRS, sind.

4. Verfahren nach einem der vorangegangenen Ansprüche, **dadurch gekennzeichnet, dass** zusätzlich zu der gleichzeitigen Ableitung der Bereitschaftspotenziale und der evozierten Potenziale auch die Aktivität der Zielmuskulatur abgeleitet wird, wobei durch eine Oberflächenableitung aus den für die Reaktion erforderlichen Muskeln die Muskelaktivität gemessen wird.

5. Verfahren nach Anspruch 1, **dadurch gekennzeichnet, dass** die Absolutwerte der Reaktionszeiten und deren Varianzen mittels eines Computers auch in Bezug auf die Aktivität der Zielmuskulatur gesetzt werden.

6. Verfahren nach Anspruch 1, **dadurch gekennzeichnet, dass** der Vergleich mit den bekannten Daten aus den klinischen und/oder statistisch validierten Untersuchungen an bewusst verzögert und mit voller Leistungsbereitschaft reagierenden gesunden Probanden sowie an hirngeschädigten und psychisch kranken Probanden erfolgt.

7. Verfahren nach einem der vorangegangenen Ansprüche, **dadurch gekennzeichnet, dass** die Ableitung an mindestens acht Orten erfolgt.

8. Verwendung des Verfahrens nach einem der Ansprüche 1 bis 7 zur Bewertung der Anstrengungsbereitschaft von Probanden in testpsychologischen Untersuchungen im entschädigungsrelevanten Kontext.

## Claims

1. Method for testing the authenticity of the reaction time achieved by a test subject, **characterized in that** a stimulus device, a reaction device, a reaction time measuring instrument, an EEG and a computer are provided and a stimulus is given to the test subject by means of the stimulus device by deriving evoked potentials from his brain wave curves measured by means of the EEG or by visualizing his brain areas by means of functional imaging methods such as near-infrared spectroscopy or fMRI (functional magnetic resonance tomography) and the testing subject is simultaneously given a reaction time task with the reaction device and the reaction time of the test subject is measured with the reaction device by deriving his readiness potentials, wherein by deriving the evoked potentials from the brain wave curve or displaying the activities of the brain areas by means of functional imaging methods, it is first checked whether the stimulus has entered the subject's consciousness and wherein the evaluation by means of the computer is carried out in such a way that the absolute values of the reaction times and their variances are set in relation to the readiness potentials and the readiness potentials are compared with the values known from other investigations in order to check the authenticity of the reaction times.

2. Method according to claim 1, **characterized in that** the evoked potentials are visually evoked potentials (VEP), acoustically evoked brainstem potentials (AEBP), somatosensitive evoked potentials (SEP) including potentials evoked by tactile means or potentials evoked by olfactory means.

3. Method according to claim 1, **characterized in that** the imaging methods are functional magnetic resonance tomography (fMRT) or near-infrared spectroscopy (NIRS) .

4. Method according to one of the preceding claims, **characterized in that**, in addition to the simultaneous derivation of the readiness potentials and the evoked potentials, the activity of the target muscles is also derived, the muscle activity being measured by a surface derivation from the muscles required for the reaction.

5. Method according to claim 1, **characterized in that** the absolute values of the reaction times and their variances are also set in relation to the activity of the target muscles by means of a computer.

6. Method according to claim 1, **characterized in that** the comparison is made with the known data from the clinical and/or statistically validated investigations on healthy test subjects reacting with a conscious delay and with full willingness to perform, as well as on brain-damaged and mentally ill test subjects.

7. Method according to one of the preceding claims, **characterized in that** the discharge takes place at at least eight locations.

8. Use of the method according to any one of claims 1 to 7 for assessing the willingness of test subjects to exert themselves in test psychological examinations in a compensation-relevant context.

## Revendications

1. Procédé destiné à vérifier l'authenticité du temps de réaction obtenu par un sujet, **caractérisé en ce que** sont prévus un appareil de stimulation, un dispositif de réaction, un appareil de mesure de temps de réaction, un EEG et un ordinateur, et **en ce qu'**un stimulus est appliqué au sujet au moyen de l'appareil de stimulation en dérivant des potentiels évoqués à partir de ses ondes électriques cérébrales mesurées au moyen de l'EEG ou en représentant ses zones cérébrales au moyen de procédés d'imagerie fonctionnelle comme la spectroscopie dans le proche infrarouge ou la tomographie fonctionnelle par résonance magnétique, et **en ce que**, de manière simultanée, le sujet se voit attribuer par le dispositif de réaction une tâche de temps de réaction, et que le temps de réaction du sujet est mesuré au moyen de l'appareil de mesure de temps de réaction en dérivant ses potentiels de disponibilité, une vérification étant tout d'abord effectuée pour savoir si le stimulus est bien parvenu jusqu'au conscient du sujet en dérivant les potentiels évoqués à partir de l'onde électrique cérébrale ou en représentant les activités des zones cérébrales au moyen de procédés d'imagerie fonctionnelle, et l'évaluation étant effectuée au moyen de l'ordinateur de telle sorte que les valeurs absolues des temps de réaction et leurs variances sont mises en relation avec les potentiels de disponibilité et que lesdits potentiels de disponibilité sont comparés aux valeurs connues issues d'autres études afin de vérifier l'authenticité des temps de réaction.

2. Procédé selon la revendication 1, **caractérisé en ce que** les potentiels évoqués sont des potentiels évoqués visuels, PEV, des potentiels évoqués acoustiques du tronc cérébral, PEATC, des potentiels évoqués somesthésiques, PES, y compris des potentiels évoqués tactiles ou des potentiels évoqués olfactifs.

3. Procédé selon la revendication 1, **caractérisé en ce que** les procédés d'imagerie utilisés sont l'imagerie par tomographie fonctionnelle par résonance magnétique, ou la spectroscopie dans le proche infrarouge, NIRS.

4. Procédé selon l'une quelconque des revendications précédentes, **caractérisé en ce que**, outre la dérivation simultanée des potentiels de disponibilité et des potentiels évoqués, on procède également à la dérivation de l'activité de la musculature cible, l'activité musculaire étant mesurée au niveau des muscles responsables de la réaction par l'intermédiaire d'une dérivation de surface.

5. Procédé selon la revendication 1, **caractérisé en ce que**, au moyen d'un ordinateur, les valeurs absolues des temps de réaction et leurs variances sont également mises en relation avec l'activité de la musculature cible.

6. Procédé selon la revendication 1, **caractérisé en ce que** la comparaison est effectuée avec les données connues issues des études cliniques et/ou validées statistiquement portant sur des sujets sains présentant une disposition à la performance intacte et réagissant délibérément de manière retardée et sur des sujets souffrant de lésions cérébrales et de maladies psychiques.

7. Procédé selon l'une quelconque des revendications précédentes, **caractérisé en ce que** la dérivation est effectuée à au moins huit endroits.

8. Utilisation du procédé selon l'une quelconque des revendications 1 à 7 destiné à évaluer la disposition à l'effort de sujets dans le cadre d'examens sous forme de tests psychologiques qui sont effectués dans le contexte de l'indemnisation.
